# EUROPEAN PATENT APPLICATION

(11) **EP 4 721 739 A1**
(43) Date of publication of application: **08.04.2026**
(21) Application number: 24814537.7
(22) Date of filing: 30.05.2024
(51) Int. Cl.: A61K 31/4439, A61P 35/00, A61P 37/00, A61P 3/00, C07D 401/14, C07D 413/14, C07D 417/14

(54) **CSF-1R INHIBITOR PHARMACEUTICAL COMPOSITION, PREPARATION METHOD THEREFOR AND PHARMACEUTICAL USE THEREOF**

(30) Priority: 02.06.2023 CN 202310654505
(71) Applicant: Abbisko Therapeutics Co., Ltd., Shanghai 201203 (CN)
(72) Inventor: GAO, Min, Shanghai 201203 (CN); ZHANG, Zhen, Shanghai 201203 (CN); YU, Hongping, Shanghai 201203 (CN)
(74) Representative: Maiwald GmbH
(86) International application number: PCT/CN2024/096336
(87) International publication number: WO 2024/245340

(57) **Abstract**

A CSF-1R inhibitor pharmaceutical composition, a preparation method therefor and a pharmaceutical use thereof. The CSF-1R inhibitor composition comprises a free base of a compound of formula (I) having the following structure or an acid salt of the compound as an active ingredient. A suitable diluent and other pharmaceutically acceptable carriers are used, and the flowability and the color uniformity of the active pharmaceutical ingredient powder are improved by adjusting the prescription components of a pharmaceutical preparation, so as to obtain a prescription and a pharmaceutical preparation.

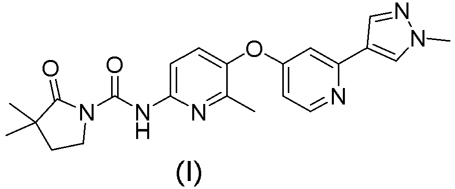

## Description

### Technical Field

The present invention belongs to the field of drug development, and in particular relates to a CSF-1R inhibitor pharmaceutical composition, a preparation method therefor and pharmaceutical use thereof.

### Background

The full name of CSF-1R (cFMS) is colony stimulating factor-1 receptor. CSF-1R belongs to the same family of three growth hormone receptors as cKIT, FLT3, and PDGFR-a&b. This receptor is a membrane protein expressed on the surface of macrophages and monocytes. Its extracellular segment can bind to macrophage colony-stimulating factor, and its intracellular tyrosine kinase can activate the downstream cell growth and proliferation signaling pathways of macrophages and monocytes, including MAPK, PI3K, etc. Therefore, the CSF-1R signaling pathway has an important influence on the development and differentiation of macrophages and monocytes, as well as the physiological functions of tumor-associated macrophages (TAM).

With the progress of tumor immunotherapy in recent years, tumor-associated macrophages (TAMs) and myeloid-derived suppressor cells (MDSCs) are considered to be directly related to the formation of the immunosuppressive microenvironment within the tumor and angiogenesis that supports tumor growth. At the same time, clinical studies have shown that the content of TAM is negatively correlated with the prognosis of cancer patients. Pharmacological experiments in mice have shown that inhibiting the CSF-1R signaling pathway can significantly reduce the number of macrophages in the tumor that suppress the immune system and increase the content of CD8-positive T cells. These experimental results suggest that CSF-1R small molecule inhibitors may reverse the immunosuppressive environment within the tumor, promote the activation of immune system, and prolong the lives of cancer patients.

Abbisko Therapeutics Co., Ltd. has developed a small molecule compound with CSF-1R inhibitory effects (WO2018214867A1, international publication date: November 29, 2018) during the long-term research process. Representative compound is as follows: 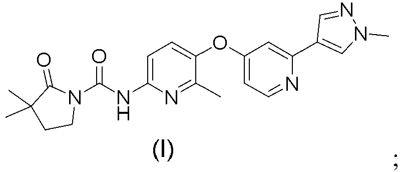 and the Chinese name is 3,3-dimethyl-N-(6-methyl-5-((2-(1-methyl-1H-pyrazol-4-yl)pyridin- 4-yl)oxy)pyridin-2-yl)-2-oxopyrrolidine-1-carboxamide (referred to as the compound of formula (I) or the free base of the compound of formula (I)). This compound can significantly enhance the inhibitory effect on the CSF-1R target and the selectivity for other kinase receptors, thereby increasing the therapeutic window and reducing clinical toxic side effects. It can be used for the targeted treatment of tumors such as lung cancer, breast cancer, prostate cancer, ovarian cancer, cervical cancer, melanoma, pancreatic cancer, head and neck cancer, glioma, and tendon sheath giant cell tumor.

However, during the later pharmaceutical research process, it was found that the free base of the compound of formula (I) exhibits poor flowability and low bulk density, failing to meet the requirements of GMP production. There is a risk of large differences in the filling amount when filling capsules, which can not meet the requirements of capsule filling. Therefore, there is an urgent need to develop a pharmaceutical composition and preparation process to overcome the above technical defects and meet the needs of clinical research and drug marketing of the compound of formula (I).

### Summary of the Invention

The purpose of the present invention is to provide a CSF-1R inhibitor pharmaceutical composition to solve the problem of drug accessibility and meet the needs of clinical research and drug marketing of the compound of formula (I).

In order to solve the problems existing in the prior art, the inventors adopt suitable diluents, optimize the types and proportions of drug carriers such as glidants, lubricants and disintegrants, and adjust the prescription ingredients of the drug preparation to improve the flowability and color uniformity of the active pharmaceutical ingredient powder. They also research and develop a prescription with a dissolution effect that meets clinical needs. All physicochemical properties meet clinical requirements. And they further obtain a prescription with good discharge and/or capsule filling uniformity. This prescription and preparation process thereof are suitable for industrial production and can meet the needs of clinical research and drug marketing of the compound of formula (I).

The first aspect of the present invention provides a pharmaceutical composition comprising a free base or an acid salt of a compound of formula (I) as an active ingredient, a pharmaceutically acceptable diluent, and other pharmaceutically acceptable carrier, wherein the other pharmaceutically acceptable carrier is one or more of a glidant, a lubricant, and a disintegrant,

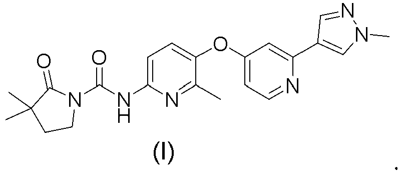

As a preferred embodiment, the acid salt of the compound of formula (I) is selected from hydrochloride, sulfate, hydrobromide, hydrofluoride, hydroiodide, phosphate, acetate, dichloroacetate, trichloroacetate, trifluoroacetate, benzenesulfonate, p-toluenesulfonate, 4-chlorobenzenesulfonate, 1,5-naphthalene disulfonate, naphthalene-2-sulfonate, ethane-1,2-disulfonate, methanesulfonate, ethanesulfonate, benzoate, decanoate, hexanoate, caprylate, cinnamate, citrate, cyclohexanesulfamate, camphorsulfonate, aspartate, camphorate, gluconate, glucuronate, glutamate, isoascorbate, lactate, aspartate, malate, mandelate, pyroglutamate, tartrate, lauryl sulfate, dibenzoyltartrate, formate, fumarate, galactonate, gentisate, acetohydroxamate, malonate, succinate, glutarate, adipate, sebacate, 2-ketoglutarate, glycolate, hippurate, isethionate, lactobionate, ascorbate, aspartate, laurate, camphorate, maleate, nicotinate, oleate, orotate, oxalate, palmitate, pamoate, propionate, 4-acetamidobenzoate, 4-aminobenzoate, salicylate, 4-amino salicylate, 2,5-dihydroxybenzoate, 1-hydroxy-2-naphthoate, stearate, thiocyanate, undecylenate, or succinate.

As a further preferred embodiment, the acid salt of the compound of formula (I) is selected from hydrochloride, sulfate, phosphate, methanesulfonate, citrate, malate, fumarate or tartrate.

As a preferred embodiment, the active ingredient is also an anhydrate, hydrate or solvate of the free base or acid salt of the compound of formula (I).

As a further preferred embodiment, the active ingredient is a solvate of the free base or acid salt of the compound of formula (I), and the solvent is selected from alcohols, chloroalkanes, ketones, ethers, cyclic ethers, esters, alkanes, cycloalkanes, benzenes, amides, sulfoxides or a mixture thereof, or an aqueous solution thereof.

As a further preferred embodiment, the solvent is selected from methanol, ethanol, n-propanol, isopropanol, dichloromethane, acetonitrile, acetone, 1,4-dioxane, tetrahydrofuran, N,N-dimethylformamide, ethyl acetate, isopropyl acetate, methyl tert-butyl ether, 2-methoxyethyl ether or a mixture thereof, or an aqueous solution thereof.

As a further preferred embodiment, the active ingredient is a hydrate of the free base or acid salt thereof, and each molecule of the hydrate contains 1 to 3 water molecules.

As a further preferred embodiment, the active ingredient is an anhydrate or hydrate of the free base or acid salt thereof.

As a further preferred embodiment, the active ingredient is an anhydrate of the free base or acid salt thereof.

As a further preferred embodiment, the active ingredient is a hydrate of the free base or acid salt thereof, and each molecule of the hydrate contains one water molecule.

As a preferred embodiment, the content of the active ingredient is calculated as the hydrochloride monohydrate of the compound of formula (I), and the pharmaceutical composition contains 0.1% to 60.0% w/w of the active ingredient relative to the total weight of the composition.

As a further preferred embodiment, the content of the active ingredient is calculated as the hydrochloride monohydrate of the compound of formula (I), and the pharmaceutical composition contains 1.0% to 40.0% w/w of the active ingredient relative to the total weight of the composition.

As a further preferred embodiment, the content of the active ingredient is calculated as the hydrochloride monohydrate of the compound of formula (I), and the pharmaceutical composition contains 3.0% to 32.0% w/w of the active ingredient relative to the total weight of the composition.

As a preferred embodiment, the diluent is one or more of lactose, lactose hydrate and microcrystalline cellulose.

As a further preferred embodiment, the pharmaceutical composition contains 40.0% to 99.9% w/w of diluent relative to the total weight of the composition.

As a further preferred embodiment, the pharmaceutical composition contains 50.0% to 94.0% w/w of diluent relative to the total weight of the composition.

As a further preferred embodiment, the pharmaceutical composition comprises 60.0% to 92.0% w/w of diluent relative to the total weight of the composition.

As a further preferred embodiment, the diluent is a mixture of lactose monohydrate and microcrystalline cellulose, and the mass ratio w/w of the lactose monohydrate to the microcrystalline cellulose is 1:5 to 5:1.

As a further preferred embodiment, the mass ratio w/w of the lactose monohydrate to the microcrystalline cellulose is 1:3 to 3:1.

As a further preferred embodiment, the mass ratio w/w of the lactose monohydrate to the microcrystalline cellulose is 1:1 to 3:1.

As a further preferred embodiment, the lactose monohydrate is lactose monohydrate T80 or lactose monohydrate 316; and the microcrystalline cellulose is microcrystalline cellulose PH102 or microcrystalline cellulose PH101.

As a further preferred embodiment, the diluent is a mixture of lactose monohydrate T80 and microcrystalline cellulose PH102, and the mass ratio w/w of the lactose monohydrate T80 and microcrystalline cellulose PH102 is 1:5 to 5:1.

As a further preferred embodiment, the mass ratio w/w of the lactose monohydrate T80 and the microcrystalline cellulose PH102 is 1:3 to 3:1.

As a further preferred embodiment, the mass ratio w/w of the lactose monohydrate T80 and the microcrystalline cellulose PH102 is 1:1 to 3:1; more preferably 2:1 or 3:1.

As a further preferred embodiment, the diluent is a mixture of lactose monohydrate T80 and microcrystalline cellulose PH101, and the mass ratio w/w of the lactose monohydrate T80 and microcrystalline cellulose PH101 is 1:5 to 5:1.

As a further preferred embodiment, the mass ratio w/w of the lactose monohydrate T80 and the microcrystalline cellulose PH101 is 1:3 to 3:1.

As a further preferred embodiment, the mass ratio w/w of the lactose monohydrate T80 and the microcrystalline cellulose PH101 is 1:1 to 3:1; more preferably 2:1 or 3:1.

As a further preferred embodiment, the diluent is a mixture of lactose monohydrate 316 and microcrystalline cellulose PH102, and the mass ratio w/w of the lactose monohydrate 316 and microcrystalline cellulose PH102 is 1:5 to 5:1.

As a further preferred embodiment, the mass ratio w/w of the lactose monohydrate 316 and the microcrystalline cellulose PH102 is 1:3 to 3:1.

As a further preferred embodiment, the mass ratio w/w of the lactose monohydrate 316 and the microcrystalline cellulose PH102 is 1:1 to 3:1; more preferably 2:1 or 3:1.

As a further preferred embodiment, the diluent is a mixture of lactose monohydrate 316 and microcrystalline cellulose PH101, and the mass ratio w/w of the lactose monohydrate 316 and microcrystalline cellulose PH101 is 1:5 to 5:1.

As a further preferred embodiment, the mass ratio w/w of the lactose monohydrate 316 and the microcrystalline cellulose PH101 is 1:3 to 3:1.

As a further preferred embodiment, the mass ratio w/w of the lactose monohydrate 316 and the microcrystalline cellulose PH101 is 1:1 to 3:1; more preferably 2:1 or 3:1.

As a preferred embodiment, the glidant is one or more of colloidal silicon dioxide, precipitated silicon dioxide and talc.

As a further preferred embodiment, the glidant is colloidal silicon dioxide.

As a further preferred embodiment, the glidant is colloidal silicon dioxide 200.

As a preferred embodiment, the glidant is one or more of colloidal silicon dioxide, precipitated silicon dioxide and talc, and the pharmaceutical composition contains 0.1% to 10.0% w/w of the glidant relative to the total weight of the composition.

As a further preferred embodiment, the glidant is colloidal silicon dioxide, and the pharmaceutical composition contains 0.5% to 5.0% w/w of the glidant relative to the total weight of the composition.

As a further preferred embodiment, the glidant is colloidal silicon dioxide, and the pharmaceutical composition contains 1.0% to 3.0% w/w of the glidant relative to the total weight of the composition.

As a further preferred embodiment, the glidant is colloidal silicon dioxide, and the pharmaceutical composition contains 1.0% to 2.0% w/w of the glidant relative to the total weight of the composition.

As a preferred embodiment, the glidant is colloidal silicon dioxide 200, and the pharmaceutical composition contains 0.1% to 10.0% w/w of the glidant relative to the total weight of the composition.

As a further preferred embodiment, the glidant is colloidal silicon dioxide 200, and the pharmaceutical composition contains 0.5% to 5.0% w/w of the glidant relative to the total weight of the composition.

As a further preferred embodiment, the glidant is colloidal silicon dioxide 200, and the pharmaceutical composition contains 1.0% to 3.0% w/w of the glidant relative to the total weight of the composition.

As a further preferred embodiment, the glidant is colloidal silicon dioxide 200, and the pharmaceutical composition contains 1.0% to 2.0% w/w of the glidant relative to the total weight of the composition.

As a preferred embodiment, the lubricant is one or more of magnesium stearate, sodium stearyl fumarate, glyceryl dibehenate, colloidal silicon dioxide, talc, other hydrogenated vegetable oils and triglyceride, and the pharmaceutical composition contains 0.1% to 5.0% w/w of the lubricant relative to the total weight of the composition.

As a further preferred embodiment, the lubricant is one or more of magnesium stearate, sodium stearyl fumarate, and glyceryl dibehenate, and the lubricant is contained in an amount of 0.5% to 2.0% w/w relative to the total weight of the composition.

As a preferred embodiment, the lubricant is magnesium stearate or sodium stearyl fumarate, and the pharmaceutical composition contains 0.1% to 5.0% w/w of the lubricant relative to the total weight of the composition.

As a further preferred embodiment, the lubricant is magnesium stearate or sodium stearyl fumarate, and the lubricant is contained in an amount of 0.5% to 2.0% w/w relative to the total weight of the composition.

As a further preferred embodiment, the lubricant is magnesium stearate or sodium stearyl fumarate, and the lubricant is contained in an amount of 0.5% to 1.5% w/w relative to the total weight of the composition.

As a preferred embodiment, the disintegrant is one or more of croscarmellose sodium, cross-linked polyvinyl pyrrolidone, sodium starch glycolate and analogue thereof.

As a further preferred embodiment, the disintegrant is croscarmellose sodium.

As a preferred embodiment, the disintegrant is one or more of croscarmellose sodium, cross-linked polyvinyl pyrrolidone, sodium starch glycolate and analogue thereof, and the pharmaceutical composition contains 0.1% to 10.0% w/w of the disintegrant relative to the total weight of the composition.

As a preferred embodiment, the disintegrant is croscarmellose sodium, and the pharmaceutical composition contains 0.1% to 10.0% w/w of the disintegrant relative to the total weight of the composition.

As a further preferred embodiment, the disintegrant is croscarmellose sodium, and the pharmaceutical composition comprises 0.5% to 5.0% w/w of the disintegrant relative to the total weight of the composition.

As a further preferred embodiment, the disintegrant is croscarmellose sodium, and the pharmaceutical composition contains 1.0% to 5.0% w/w of the disintegrant relative to the total weight of the composition.

As a preferred embodiment, the pharmaceutical composition comprises an active ingredient and a diluent, and the mass ratio w/w is (1.0-50.0): (50.0-94.0), and the content of the active ingredient is calculated as the hydrochloride monohydrate of the compound of formula (I); the diluent is lactose monohydrate and microcrystalline cellulose, and the mass ratio w/w is 1:1-3:1.

As a preferred embodiment, the pharmaceutical composition comprises an active ingredient and a diluent, and the mass ratio w/w is (1.0-50.0): (60.0-92.0), and the content of the active ingredient is calculated as the hydrochloride monohydrate of the compound of formula (I); the diluent is lactose monohydrate T80 and microcrystalline cellulose PH102, and the mass ratio w/w is 1:1-3:1.

As a preferred embodiment, the pharmaceutical composition comprises an active ingredient and a diluent, and the mass ratio w/w is (1.0-50.0): (60.0-92.0), and the content of the active ingredient is calculated as the hydrochloride monohydrate of the compound of formula (I); the diluent is lactose monohydrate T80 and microcrystalline cellulose PH101, and the mass ratio w/w is 1:1-3:1.

As a preferred embodiment, the pharmaceutical composition comprises an active ingredient and a diluent, and the mass ratio w/w is (1.0-50.0): (60.0-92.0), and the content of the active ingredient is calculated as the hydrochloride monohydrate of the compound of formula (I); the diluent is lactose monohydrate 316 and microcrystalline cellulose PH102, and the mass ratio w/w is 1:1-3:1.

As a preferred embodiment, the pharmaceutical composition comprises an active ingredient and a diluent, and the mass ratio w/w is (1.0-50.0): (60.0-92.0), and the content of the active ingredient is calculated as the hydrochloride monohydrate of the compound of formula (I); the diluent is lactose monohydrate 316 and microcrystalline cellulose PH101, and the mass ratio w/w is 1:1-3:1.

As a further preferred embodiment, the pharmaceutical composition further comprises colloidal silicon dioxide as a glidant, and the content of colloidal silicon dioxide is 0.5% to 5.0% w/w relative to the total weight of the composition.

As a further preferred embodiment, the content of the glidant is 1.0% to 3.0% w/w relative to the total weight of the composition.

As a further preferred embodiment, the content of the glidant is 1.0% to 2.0% w/w relative to the total weight of the composition.

As a further preferred embodiment, the pharmaceutical composition further comprises magnesium stearate or sodium stearyl fumarate as a lubricant, with a content of 0.5% to 2.0% w/w relative to the total weight of the composition.

As a further preferred embodiment, the content of the lubricant is 0.5% to 1.5% w/w relative to the total weight of the composition.

As a further preferred embodiment, the pharmaceutical composition further comprises croscarmellose sodium as a disintegrant, and the content thereof is 0.5% to 5.0% w/w relative to the total weight of the composition.

As a further preferred embodiment, the content of the disintegrant is 1.0% to 5.0% w/w relative to the total weight of the composition.

As a preferred embodiment, the sum of the contents of each component in the pharmaceutical composition is 100 wt%. As a further preferred embodiment, the total content of the active ingredient, diluent, glidant, lubricant and disintegrant is 100 wt%.

As a further preferred embodiment, the active ingredient, diluent, glidant, lubricant and disintegrant are as described above.

As a further preferred embodiment, in the pharmaceutical composition, the active ingredient is the hydrochloride monohydrate of the compound of formula (I), the diluent is microcrystalline cellulose PH102 and lactose monohydrate T80, the glidant is colloidal silicon dioxide 200, the lubricant is magnesium stearate, and the disintegrant is croscarmellose sodium.

As a further preferred embodiment, in the pharmaceutical composition, the active ingredient is the hydrochloride monohydrate of the compound of formula (I), the diluent is microcrystalline cellulose PH101 and lactose monohydrate 316, the glidant is colloidal silicon dioxide 200, the lubricant is magnesium stearate, and the disintegrant is croscarmellose sodium.

As a preferred embodiment, the pharmaceutical composition is in a capsule form.

As a further preferred embodiment, the unit dosage form of the capsule contains 1 mg to 500 mg of active ingredient, and the content of the active ingredient is calculated as the free base of the compound of formula (I).

As a further preferred embodiment, the unit dosage form of the capsule contains 1 mg to 200 mg of active ingredient, and the content of the active ingredient is calculated as the free base of the compound of formula (I).

As a further preferred embodiment, the unit dosage form of the capsule contains 1 mg, 5 mg, 25 mg, 50 mg, 75 mg, 100 mg, 150 mg or 200 mg of active ingredient, and the content of the active ingredient is calculated as the free base of the compound of formula (I).

The second aspect of the present invention provides a method for preparing a pharmaceutical composition, comprising the following steps:
step A: pre-treating the free base or acid salt of the compound of formula (I) and the glidant by sieving, either separately or in combination; 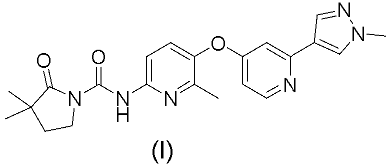
pre-treating the diluent, disintegrant and lubricant by sieving, either separately or in combination;
step B: uniformly mixing the mixture of free base or acid salt of the compound of formula (I) and the glidant prepared in step A with the pretreated diluent and disintegrant, and placing them in a hopper mixer for mixing;
step C: uniformly mixing the mixed material prepared in step B and the pretreated lubricant, and placing them in a mixing barrel for mixing;

As a preferred embodiment, the preparation method further comprises step D: filling the total mixed powder prepared in step C into capsules.

As a preferred embodiment, the preparation method comprises: mixing the free base or acid salt of the compound of formula (I) and the glidant and then pre-treating the mixture through a 50-80 mesh sieve; and/or, pre-treating the diluent independently through a 30-80 mesh sieve; and/or, pre-treating the disintegrant independently through a 30-50 mesh sieve; and/or, pre-treating the lubricant independently through a 20-40 mesh sieve.

As a preferred embodiment, in step A of the preparation method, the free base or acid salt of the compound of formula (I) is pulverized.

The third aspect of the present invention relates to use of the above-mentioned pharmaceutical composition in the manufacture of CSF-1R inhibitor drug.

The fourth aspect of the present invention relates to use of the above-mentioned pharmaceutical composition in the manufacture of a medicament for treating a tumor, autoimmune disease, metabolic disease or metastatic disease associated with CSF1-R.

As a preferred embodiment, the tumor is cancer.

As a preferred embodiment, the tumor, autoimmune disease, metabolic disease or metastatic disease associated with CSF1-R is a drug for ovarian cancer, pancreatic cancer, prostate cancer, lung cancer, breast cancer, kidney cancer, liver cancer, cervical cancer, bone metastatic cancer, papillary thyroid cancer, non-small cell lung cancer, colon cancer, gastrointestinal stromal tumor, solid tumor, melanoma, mesothelioma, glioblastoma, osteosarcoma, multiple myeloma, hyperproliferative disease, metabolic disease, neurodegenerative disease, metastasis of primary tumor site, myeloproliferative disease, leukemia, rheumatism arthritis, rheumatoid arthritis, osteoarthritis, multiple sclerosis, autoimmune nephritis, lupus, Crohn's disease, asthma, chronic obstructive pulmonary disease, osteoporosis, hypereosinophilic syndrome, mastocytosis or mast cell leukemia.

The fifth aspect of the present invention relates to the above-mentioned pharmaceutical composition for use as a medicament for treating a tumor, autoimmune disease, metabolic disease or metastatic disease associated with CSF1-R.

The present invention also provides a method for inhibiting CSF-1R activity, comprising administering an effective therapeutic amount of the above-mentioned pharmaceutical composition to a patient in need of treatment.

The present invention also provides a method for treating ovarian cancer, pancreatic cancer, prostate cancer, lung cancer, breast cancer, kidney cancer, liver cancer, cervical cancer, bone metastatic cancer, papillary thyroid cancer, non-small cell lung cancer, colon cancer, gastrointestinal stromal tumor, solid tumor, melanoma, mesothelioma, glioblastoma, osteosarcoma, multiple myeloma, hyperproliferative disease, metabolic disease, neurodegenerative disease, metastasis of primary tumor site, myeloproliferative disease, leukemia, rheumatism arthritis, rheumatoid arthritis, osteoarthritis, multiple sclerosis, autoimmune nephritis, lupus, Crohn's disease, asthma, chronic obstructive pulmonary disease, osteoporosis, hypereosinophilic syndrome, mastocytosis or mast cell leukemia associated with CSF1-R, comprising administering to a patient in need of treatment an effective therapeutic amount of the above-mentioned pharmaceutical composition.

### Detailed Description of the Invention

### 1. Specific definition

Detailed Description: Unless otherwise stated or specifically indicated, the following terms used in the specification and claims have the following meanings.

The term "pharmaceutical composition" refers to a mixture containing one or more compounds described herein or their physiologically/pharmaceutically acceptable crystalline forms, salt forms, or prodrugs, and other chemical components, as well as other components such as physiologically/pharmaceutically acceptable carriers. The purpose of a pharmaceutical composition is to facilitate administration to an organism, facilitate the absorption of the active ingredients, and thus exert biological activity.

The term "free base of a compound of formula (I)" refers to a compound of formula (I),

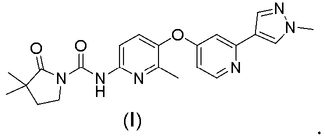

The term "active ingredient" refers to a free base of the compound of formula (I) or acid salt of the compound of formula (I); the active ingredient is preferably an anhydrate, hydrate or solvate of the free base or acid salt of the compound of formula (I); the solvent refers to an organic solvent selected from alcohols, chloroalkanes, ketones, ethers, cyclic ethers, esters, alkanes, cycloalkanes, benzenes, amides, sulfoxides or a mixture thereof, or an aqueous solution thereof. Preferably, the solvent is selected from methanol, ethanol, n-propanol, isopropanol, dichloromethane, acetonitrile, acetone, 1,4-dioxane, tetrahydrofuran, N,N-dimethylformamide, ethyl acetate, isopropyl acetate, methyl tert-butyl ether, 2-methoxyethyl ether or a mixture thereof, or an aqueous solution thereof.

The pharmaceutical composition of the present invention may include one or more pharmaceutically acceptable carriers, including but not limited to diluent, glidant, lubricant, disintegrant, binder, stabilizer, buffer, adjuvant, carrier, emulsifier, viscosity regulator, surfactant, preservative, flavoring agent or coloring agent. Medically acceptable excipients can be found in the examples described in R.C. Rowe, Paul J. Sheskey, The Handbook of Pharmaceutical Excipients.

As used herein, the term "diluent" refers to any pharmaceutically acceptable substance or composition added to a composition to increase volume. Suitable diluents include, but are not limited to, lactose (e.g., lactose monohydrate, anhydrous lactose, or amorphous lactose), microcrystalline cellulose (e.g., PH101, PH102, PH301, PH302, PH200, Ceolus KG-802, Ceolus KG-1000, Ceolus UF-711, Ceolus UF-702, Prosolv SMCC50, SMCC90 and the like), silicified microcrystalline cellulose, mannitol, and dicalcium phosphate.

As used herein, the term "glidant" refers to any pharmaceutically acceptable substance or composition added to a composition to increase flowability. Suitable glidants include, but are not limited to, colloidal silicon dioxide, precipitated silicon dioxide, and/or talc.

As used herein, the term "lubricant" refers to any pharmaceutically acceptable agent added to a composition to reduce surface friction, lubricate the surface of particles, reduce the tendency of static charge accumulation, and/or reduce the friability of particles. Thus, the lubricant may act as an anti-agglomeration agent. Conventional lubricants include stearic acid and related compounds, such as magnesium stearate and sodium stearyl fumarate. Alternative lubricants include glyceryl dibehenate, colloidal silicon dioxide, talc, other hydrogenated vegetable oils and triglyceride. Examples of suitable alternative lubricants include, but are not limited to, glyceryl dibehenate.

As used herein, the term "disintegrant" refers to a substance added to a composition to help it break up (disintegrate) and release the pharmaceutical agent. Examples of disintegrants include, but are not limited to, non-sugar water-soluble polymers such as cross-linked polyvinyl pyrrolidone. Other disintegrants that may be used include, for example, croscarmellose sodium, sodium starch glycolate, and analogue thereof, see, for example, Khattab (1992) J. Pharm. Pharmacol. 45: 687-691.

The terms "lactose monohydrate T80" and "lactose monohydrate 316" refer to block particles obtained by granulating lactose monohydrate powder. This specification indicates roughness and a large specific surface area.

The term "lactose monohydrate FLOWLAC^{®}100" refers to spherical particles with a porous surface structure obtained by spray drying an aqueous lactose solution.

The term "silicified microcrystalline cellulose" refers to a substance prepared by blending microcrystalline cellulose and colloidal silicon dioxide in water followed by drying. On a dry weight basis, it typically contains 94.0-100% microcrystalline cellulose.

### 2. Experimental instruments and materials

### 2.1 Instruments and Equipments

| **Name** | **Model** | **Manufacturer** |
|---|---|---|
| Bulk Hopper Mixer | HSD50 | Zhejiang Canaan Technology Co., Ltd. |
| Dissolution Tester | 708-DS | Agilent Technologies Co., Ltd. |
| Small Grinder | ZN-04AL | Beijing Kingshilihe Technology Development Co., Ltd. |
| Capsule Filling Machine | NJP-200C-2 | Shandong Xinma Pharmaceutical Equipment Co., Ltd. |

### 2.2 Material Information

| **Material Name** | **Supplier** |
|---|---|
| Active Pharmaceutical Ingredient (API) (hydrochloride monohydrate of a compound of formula (I)) | Abbisko |
| Lactose Monohydrate 316 | KERRY, USP |
| Lactose Monohydrate T80 | MEGGLE, Germany |
| Lactose Monohydrate FLOWLAC^{®}100 | MEGGLE, Germany |
| Microcrystalline Cellulose PH102 | FMC, USA |
| Microcrystalline Cellulose PH101 | FMC, USA |
| croscarmellose sodium | JRS, Germany |
| colloidal silicon dioxide 200 | Evonik, Germany |
| Magnesium Stearate | Mallinkrodt, USA |
| empty gelatin capsule, 3#, opaque, rich yellow | Suzhou Capsule Co., Ltd. |
| high-density polyethylene bottles for oral solid medications, 75 mL | Sena Medical Packaging Materials (Kunshan) Co., Ltd. |
| child-resistant caps for oral solid medications | |

To objectively evaluate the compatibility between the active pharmaceutical ingredient (API) and the excipients used, this invention employs common excipients for oral solid preparations. These excipients are mixed with the API at varying ratios and subjected to different storage conditions. Samples are retrieved at specific time points and assessed based on appearance, moisture absorption weight gain, content, and related substances to determine the compatibility between the API and excipients. According to the excipient manual, all excipients used are commonly used excipients for oral solid preparations and have been included in the current edition of the Chinese Pharmacopoeia. The properties of each excipient are stable. This invention conducts a compatibility test on the active pharmaceutical ingredient and various excipients. The ratio of the active pharmaceutical ingredient and excipients is set according to the "Basic Technical Guidelines for Research on Chemical Drug Preparations". The excipients with larger amounts (such as microcrystalline cellulose) are mixed according to the weight ratio of the active pharmaceutical ingredient: diluent = 1:5; the excipients with smaller amounts (such as magnesium stearate) are mixed according to the weight ratio of the active pharmaceutical ingredient: lubricant = 5:1. The samples are placed under experimental conditions of high temperature (60 °C, 40 °C), high humidity (25 °C/92.5% RH), accelerated condition (40 °C/75% RH), and light exposure (visible light 4500 Lux±500Lux, near-ultraviolet light 85 µw/cm²), and samples are taken for inspection on the 0th day, the 10th day, the 30th day, and the 6th month. The samples are tested for relevant indicators (including appearance, moisture absorption weight gain, content, and related substances), and the results are compared with the control sample API.

Test results on the compatibility of the active pharmaceutical ingredient with excipients show that under high temperature 60 °C, high humidity 25 °C/92.5% RH, accelerated condition 40 °C/75% RH and light exposure, API has good compatibility with lactose monohydrate T80, lactose monohydrate 316, microcrystalline cellulose PH101, microcrystalline cellulose PH102, croscarmellose sodium, colloidal silicon dioxide 200, magnesium stearate and empty gelatin capsules.

### 3. Dissolution assay method

| | |
|---|---|
| Dissolution Apparatus | Chinese Pharmacopoeia 2020 Edition, 0931, Method 1 (Basket Method) |
| Dissolution Medium | 0.1 mol/L hydrochloric acid solution |
| Medium pH | pH 1.2 |
| Medium Volume | 900 mL |
| Medium Temperature | 37 ± 0.5 °C |
| Rotation Speed | 50 rpm |
| Sampling Time Points | 45 min (Dissolution Degree) |
| | 5 min, 15 min, 30 min, 45 min, 60 min (Dissolution Profile) |
| Sampling Volume | 5 mL |
| Filtration | 0.45 µm PES filter, discard at least 3 mL |

### 4. Determination method of content uniformity

Several capsules prepared in Example 7 of the present application are taken, and the relative content of each capsule relative to the labeled amount (100%) is measured. The mean and standard deviation (SD%) and the absolute value of the difference between the labeled amount and the mean (A) are calculated. The content uniformity CP of the pharmaceutical composition of the present application is calculated by CP = A + 2.2S (CP ≤ 15.0 meets the requirements of the Chinese Pharmacopoeia).

Chromatographic conditions: octadecylsilane bonded silica gel as the filler (Waters XBridge^{®} C18, 4.6×150 mm, 3.5 µm column); 0.1 vol% trifluoroacetic acid in water as mobile phase A, 0.1 vol% trifluoroacetic acid in acetonitrile as mobile phase B, isocratic elution with mobile phase A-mobile phase B (60:40), detection wavelength at 245 nm, flow rate at 1.0 mL/min, column temperature at 40 °C, injection volume at 10 µL, and run time at 7 minutes.

### 5. Determination of related substances

Chromatographic conditions: octadecylsilane bonded silica gel as the filler (Waters CORTECS^{®} C18+, 2.1 × 100 mm, 2.7 µm column), 0.05 vol% trifluoroacetic acid aqueous solution (pH adjusted to 3.0 with triethylamine) as mobile phase A, acetonitrile as mobile phase B, detection wavelength at 245 nm, flow rate at 0.4 mL/min, column temperature at 25 °C, and injection volume of 2 µL. Perform gradient elution according to the table below, with a run time of 24.1 minutes and a post-run time of 5 minutes.

| Time (minutes) | 0.0 | 3.0 | 7.0 | 10.0 | 17.0 | 19.0 | 24.0 | 24.1 |
|---|---|---|---|---|---|---|---|---|
| Mobile phase A (%) | 95 | 95 | 80 | 70 | 70 | 10 | 10 | 95 |
| Mobile phase B (%) | 5 | 5 | 20 | 30 | 30 | 90 | 90 | 5 |

The reagents in the examples of the present invention are known and commercially available. The reagents used are commercially available industrial-grade or analytical-grade reagents, or can be synthesized by or according to methods known in the art. In particular, the free base of the compound of formula (I) is prepared according to WO2018214867A1. The test methods not specifically described in the present invention are all performed according to conventional techniques or methods in the art and are not listed herein one by one.

The present invention is further described in detail and completely in the following examples, which are only used to illustrate specific embodiments of the present invention and should not be interpreted as limiting the scope of the present invention in any way.

### Example 1

### (Evaluation of the flowability of pharmaceutical composition)

### 1. Existing technical problems

During the pharmaceutical research process, the inventors of this application found that API had poor flowability and low bulk density, which could not meet the requirements of GMP production. There was a risk of large differences in filling volume when filling capsules, which could not meet the requirements of capsule filling.

### 2. Flowability Assessment Program

In view of the problems existing in the prior art, the inventors of the present application designed three specifications (5 mg, 25 mg and 100 mg) of pharmaceutical compositions and evaluated the flowability of the pharmaceutical compositions by examining parameters such as bulk density, Carr index and angle of repose.

According to the components and amounts listed in the table below, the inventors mixed the API and colloidal silicon dioxide 200 and sieved them. Sieve-treated lactose monohydrate FLOWLAC^{®}100 or lactose monohydrate T80, microcrystalline cellulose PH102, and croscarmellose sodium were added and mixed uniformly in a hopper mixer at 20 rpm for 15-45 minutes. Sieve-treated magnesium stearate was then added and mixed uniformly. The mixture was placed in a mixing barrel and mixed at 20 rpm for 3-6 minutes.

After preliminary process screening, the inventors finally decided to use the powder direct filling process, examined the powder properties of the prescription, and used dissolution as the product indicator.

### 3. Drug components and contents

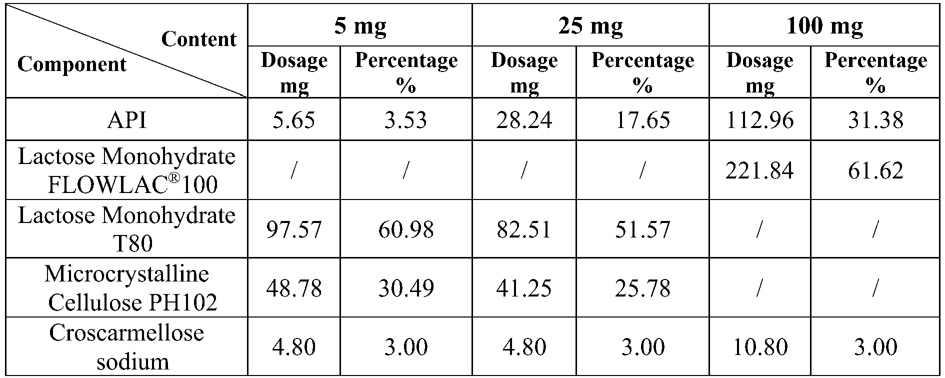

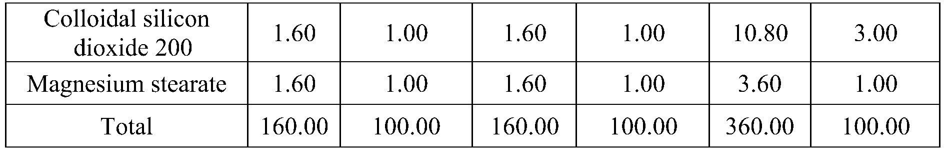

Note: The formulation specifications in this application are based on the free base of the compound of formula (I). The "API" used actually is the hydrochloride monohydrate of the compound of formula (I), with a molecular weight of 474.95 and a free base molecular weight of 420.47. The conversion factor is 1.1296. Unless otherwise specified in the following examples, the API used is the same as that in Example 1.

### 4. Powder properties data

| **Specification** | **Bulk Density (g/ml)** | **Tapped Density (g/ml)** | **Carr Index (%)** | **Angle of Repose (°)** |
|---|---|---|---|---|
| API | 0.241 | 0.596 | 59.56 | 44.63 |
| 5 mg | 0.499 | 0.688 | 27.47 | 39.77 |
| 25 mg | 0.502 | 0.717 | 29.99 | 39.46 |
| 100 mg | 0.509 | 0.746 | 31.77 | 38.15 |

From the above data, it can be seen that although the API has poor flowability and low bulk density, the bulk density of the powder is significantly improved after being prepared into a pharmaceutical composition, and the angle of repose and Carr index are significantly reduced. This can reduce the risk of large filling volume differences when filling capsules, and can meet the requirements of capsule filling, thereby facilitating the realization of the powder direct filling process.

### Example 2

### (Evaluation of the effect of diluent)

### 1. Mixing uniformity inspection

### 1.1 Drug components and contents

The inventors prepared pharmaceutical compositions 2-1 to 2-4 according to the drug components and contents described in the following table and referring to the method of Example 1.

| **Test No.** | **2-1** | | **2-2** | | **2-3** | | **2-4** | |
|---|---|---|---|---|---|---|---|---|
| **Component and Content** | **Dosage mg** | **Percentage %** | **Dosage mg** | **Percentage %** | **Dosage mg** | **Percentage %** | **Dosage mg** | **Percentage %** |
| API | 112.96 | 31.38 | 112.96 | 28.24 | 112.96 | 31.38 | 28.24 | 17.65 |
| Lactose Monohydrate FLOWLAC^{®}100 | 221.84 | 61.62 | / | / | / | / | / | / |
| Lactose Monohydrate T80 | / | / | 259.04 | 64.76 | 110.92 | 30.81 | / | / |
| Lactose Monohydrate 316 | / | / | / | / | / | / | 82.51 | 51.57 |
| Microcrystalline Cellulose PH101 | / | / | / | / | / | / | 41.25 | 25.78 |
| Microcrystalline Cellulose PH102 | / | / | / | / | 110.92 | 30.81 | / | / |
| croscarmellose sodium | 10.80 | 3.00 | 12.00 | 3.00 | 10.80 | 3.00 | 4.80 | 3.00 |
| colloidal silicon dioxide 200 | 10.80 | 3.00 | 12.00 | 3.00 | 10.80 | 3.00 | 1.60 | 1.00 |
| Magnesium stearate | 3.60 | 1.00 | 4.00 | 1.00 | 3.60 | 1.00 | 1.60 | 1.00 |
| Total | 360.00 | 100.00 | 400.00 | 100.00 | 360.00 | 100.00 | 160.00 | 100.00 |

### 1.2 Powder properties data

| **Test No.** | **Bulk Density (g/ml)** | **Tapped Density (g/ml)** | **Carr Index (%)** | **Angle of Repose (°)** |
|---|---|---|---|---|
| 2-1 | 0.509 | 0.746 | 31.77 | 38.15 |
| 2-2 | 0.543 | 0.809 | 32.88 | 38.69 |
| 2-3 | 0.434 | 0.663 | 34.54 | 41.35 |
| 2-4 | 0.500 | 0.720 | 30.56 | 37.20 |

It can be seen from the experimental results in the above table that the pharmaceutical compositions containing lactose monohydrate FLOWLAC^{®}100, lactose monohydrate T80 and compound diluents composed of lactose monohydrate T80 and microcrystalline cellulose PH102 or lactose monohydrate 316 and microcrystalline cellulose PH101 all have acceptable powder flowability. However, during the experiment, it was found that the powder mixture of the pharmaceutical composition containing lactose monohydrate FLOWLAC^{®}100 as diluent was uneven in color and had color difference, and there was a risk of uneven powder mixing and demixing. The powder mixture of the pharmaceutical composition containing lactose monohydrate T80, lactose monohydrate 316 and/or microcrystalline cellulose PH101, microcrystalline cellulose PH102 as diluents had uniform color and no risk of demixing.

### 2. Investigation of flowability and dissolution effect

### 2.1 Drug components and contents

The inventors prepared pharmaceutical compositions 2-5 to 2-9 according to the components and contents described in the following table and referring to the method of Example 1.

| **Test No.** | **2-5** | | **2-6** | | **2-7** | |
|---|---|---|---|---|---|---|
| **Component and Content** | **Dosage mg** | **Percentage %** | **Dosage mg** | **Percentage %** | **Dosage mg** | **Percentage %** |
| API | 112.96 | 28.24 | 112.96 | 31.38 | 112.96 | 31.38 |
| Lactose Monohydrate T80 | 259.04 | 64.76 | 147.89 | 41.08 | 110.92 | 30.81 |
| Microcrystalline Cellulose PH102 | / | / | 73.95 | 20.54 | 110.92 | 30.81 |
| croscarmellose sodium | 12.00 | 3.00 | 10.80 | 3.00 | 10.80 | 3.00 |
| colloidal silicon dioxide 200 | 12.00 | 3.00 | 10.80 | 3.00 | 10.80 | 3.00 |
| Magnesium stearate | 4.00 | 1.00 | 3.60 | 1.00 | 3.60 | 1.00 |
| Total | 400.00 | 100.00 | 360.00 | 100.00 | 360.00 | 100.00 |
| | | | | | | |

| **Test No.** | **2-8** | | **2-9** | | | |
|---|---|---|---|---|---|---|
| **Component and Content** | **Dosage mg** | **Percentage %** | **Dosage mg** | **Percentage %** | | |
| API | 28.24 | 17.65 | 28.24 | 17.65 | | |
| Lactose Monohydrate 316 | 61.88 | 38.68 | 92.82 | 58.01 | | |
| Microcrystalline Cellulose PH101 | 61.88 | 38.67 | 30.94 | 19.34 | | |
| croscarmellose sodium | 4.80 | 3.00 | 4.80 | 3.00 | | |
| colloidal silicon dioxide 200 | 1.60 | 1.00 | 1.60 | 1.00 | | |
| Magnesium stearate | 1.60 | 1.00 | 1.60 | 1.00 | | |
| Total | 160.00 | 100.00 | 160.00 | 100.00 | | |

### 2.2 Powder properties data

| **Test No.** | **Bulk Density (g/ml)** | **Tapped Density (g/ml)** | **Carr Index (%)** | **Angle of Repose (°)** |
|---|---|---|---|---|
| **2-5** | 0.543 | 0.809 | 32.88 | 38.69 |
| **2-6** | 0.463 | 0.703 | 34.14 | 42.23 |
| **2-7** | 0.434 | 0.663 | 34.54 | 41.35 |
| **2-8** | 0.480 | 0.690 | 30.43 | 38.3 |
| **2-9** | 0.550 | 0.780 | 29.49 | 35.3 |

From the above experimental results, it can be seen that different pharmaceutical compositions containing lactose monohydrate T80 or lactose monohydrate 316 and/or microcrystalline cellulose PH102 or microcrystalline cellulose PH101 all have acceptable powder flowability.

### 2.3 Drug dissolution results

| **Test No.** | **Dissolution Item** | **Dissolution (%) (n=3)** | | | |
|---|---|---|---|---|---|
| | | **5 min** | **15 min** | **30 min** | **45 min** |
| **2-4** | Mean | 91.6 | 99.2 | 100.1 | 100.3 |
| | RSD% | 3.0 | 2.1 | 2.0 | 2.0 |
| **2-5** | Mean | 33 | 83 | 96 | 97 |
| | RSD% | 7.9 | 7.3 | 1.1 | 1.0 |
| **2-6** | Mean | 52 | 92 | 99 | 100 |
| | RSD% | 15.4 | 6.1 | 1.0 | 1.5 |
| **2-7** | Mean | 56 | 93 | 100 | 101 |
| | RSD% | 12.2 | 0.7 | 0.9 | 0.7 |
| **2-8** | Mean | 92.2 | 101.8 | 102.6 | 102.6 |
| | RSD% | 5.6 | 1.1 | 2.1 | 2.0 |
| **2-9** | Mean | 93.6 | 98.5 | 99.2 | 99.4 |
| | RSD% | 3.7 | 4.2 | 4.4 | 4.4 |
| Note | "Mean" refers to the average of three measurements; "RSD" refers to the relative standard deviation. The meanings of 'Mean' and "RSD" in the following examples are identical to those in this example. | | | | |

From the above experimental results, it can be seen that different pharmaceutical compositions containing lactose monohydrate T80, lactose monohydrate 316 and/or microcrystalline cellulose PH101, microcrystalline cellulose PH102 all have good dissolution behavior. The pharmaceutical composition containing a mixture of lactose monohydrate T80 and microcrystalline cellulose PH102 or a mixture of lactose monohydrate 316 and microcrystalline cellulose PH101 as a diluent has better dissolution behavior, especially when a mixture of lactose monohydrate 316 and microcrystalline cellulose PH101 is used as a diluent, the pharmaceutical composition has a dissolution of more than 90% within 5 minutes and can take effect quickly.

In particular, when the ratio of lactose monohydrate T80 to microcrystalline cellulose PH102 is 2:1 or the ratio of lactose monohydrate 316 to microcrystalline cellulose PH101 is 2:1 or 3:1, the pharmaceutical composition exhibits not only improved dissolution behavior but also higher bulk density and tapped density, making it easier to fill.

### Example 3

### (Evaluation of the effect of glidant)

### 1. Drug components and contents

The inventors prepared pharmaceutical compositions 3-1 to 3-6 according to the components and contents described in the following table and referring to the method of Example 1, and investigated the dosage of the glidant.

| **Test No.** | **3-1** | | **3-2** | | **3-3** | |
|---|---|---|---|---|---|---|
| **Component and Dosage** | **Dosage mg** | **Percentage %** | **Dosage mg** | **Percentage %** | **Dosage mg** | **Percentage %** |
| API | 112.96 | 31.38 | 112.96 | 31.38 | 112.96 | 31.38 |
| Lactose Monohydrate T80 | 152.69 | 42.41 | 150.30 | 41.75 | 147.89 | 41.08 |
| Microcrystalline Cellulose PH102 | 76.35 | 21.21 | 75.14 | 20.87 | 73.95 | 20.54 |
| croscarmellose sodium | 10.80 | 3.00 | 10.80 | 3.00 | 10.80 | 3.00 |
| colloidal silicon dioxide 200 | 3.60 | 1.00 | 7.20 | 2.00 | 10.80 | 3.00 |
| Magnesium stearate | 3.60 | 1.00 | 3.60 | 1.00 | 3.60 | 1.00 |
| Total | 360.00 | 100.00 | 360.00 | 100.00 | 360.00 | 100.00 |
| | | | | | | |

| **Test No.** | **3-4** | | **3-5** | | **3-6** | |
|---|---|---|---|---|---|---|
| **Component and Dosage** | **Dosage mg** | **Percentage %** | **Dosage mg** | **Percentage %** | **Dosage mg** | **Percentage %** |
| API | 28.24 | 17.65 | 28.24 | 17.65 | 28.24 | 17.65 |
| Lactose Monohydrate 316 | 82.51 | 51.57 | 82.51 | 51.57 | 82.51 | 51.57 |
| Microcrystalline Cellulose PH101 | 42.05 | 26.28 | 41.25 | 25.78 | 39.65 | 24.78 |
| croscarmellose sodium | 4.80 | 3.00 | 4.80 | 3.00 | 4.80 | 3.00 |
| colloidal silicon dioxide 200 | 0.80 | 0.50 | 1.60 | 1.00 | 3.20 | 2.00 |
| Magnesium stearate | 1.60 | 1.00 | 1.60 | 1.00 | 1.60 | 1.00 |
| Total | 160.00 | 100.00 | 160.00 | 100.00 | 160.00 | 100.00 |

### 2. Powder properties data

| **Test No.** | **Bulk Density (g/ml)** | **Tapped Density (g/ml)** | **Carr Index (%)** | **Angle of Repose (°)** |
|---|---|---|---|---|
| **3-1** | 0.488 | 0.742 | 34.23 | 40.36 |
| **3-2** | 0.459 | 0.705 | 34.89 | 40.62 |
| **3-3** | 0.463 | 0.703 | 34.14 | 42.23 |
| **3-4** | 0.50 | 0.71 | 29.58 | 35.7 |
| **3-5** | 0.50 | 0.72 | 30.56 | 37.2 |
| **3-6** | 0.48 | 0.69 | 30.43 | 37.5 |

### 3. Drug dissolution results

| **Test No.** | **Dissolution Item** | **Dissolution (%) (n=3)** | | | |
|---|---|---|---|---|---|
| | | **5 min** | **15 min** | **30 min** | **45 min** |
| **3-4** | Mean | 93.9 | 98.3 | 98.8 | 99.1 |
| | RSD% | 3.1 | 1.8 | 1.9 | 1.8 |
| **3-5** | Mean | 91.6 | 99.2 | 100.1 | 100.3 |
| | RSD% | 3.0 | 2.1 | 2.0 | 2.0 |
| **3-6** | Mean | 93.9 | 100.2 | 100.8 | 100.9 |
| | RSD% | 2.2 | 2.1 | 2.1 | 2.1 |

The above experimental results show that a good flow-aiding effect can be obtained when the amount of glidant is 0.5%. As the amount increases, the angle of repose increases accordingly, which is more obvious at 3%. This shows that excessive use of glidant will affect the flowability of the powder. Therefore, when the amount of colloidal silicon dioxide is 1% to 2%, the effect is better. The drug dissolution results show that the three pharmaceutical compositions with colloidal silicon dioxide in the range of 0.5% to 2.0% have faster dissolution rates and good dissolution consistency in a dissolution medium (0.1 mol/L hydrochloric acid solution) with a pH of 1.2.

### Example 4

### (Evaluation of the effect of Lubricant)

### 1. Drug components and contents

The inventors prepared pharmaceutical compositions 4-1 to 4-6 according to the components and contents described in the following table and with reference to the method of Example 1, and investigated the amount of lubricant used.

| **Test No.** | **4-1** | | **4-2** | | **4-3** | |
|---|---|---|---|---|---|---|
| **Component and Content** | **Dosage mg** | **Percentage %** | **Dosage mg** | **Percentage %** | **Dosage mg** | **Percentage %** |
| API | 112.96 | 31.38 | 112.96 | 31.38 | 112.96 | 31.38 |
| Lactose Monohydrate T80 | 153.89 | 42.75 | 152.69 | 42.41 | 151.49 | 42.08 |
| Microcrystalline Cellulose PH102 | 76.95 | 21.37 | 76.35 | 21.21 | 75.75 | 21.04 |
| croscarmellose sodium | 10.80 | 3.00 | 10.80 | 3.00 | 10.80 | 3.00 |
| colloidal silicon | 3.60 | 1.00 | 3.60 | 1.00 | 3.60 | 1.00 |
| dioxide 200 | | | | | | |
| Magnesium stearate | 1.80 | 0.50 | 3.60 | 1.00 | 5.40 | 1.50 |
| Total | 360.00 | 100.00 | 360.00 | 100.00 | 360.00 | 100.00 |
| | | | | | | |

| **Test No.** | **4-4** | | **4-5** | | **4-6** | |
|---|---|---|---|---|---|---|
| **Component and Content** | **Dosage mg** | **Percentage %** | **Dosage mg** | **Percentage %** | **Dosage mg** | **Percentage %** |
| API | 28.24 | 17.65 | 28.24 | 17.65 | 28.24 | 17.65 |
| Lactose Monohydrate 316 | 82.51 | 51.57 | 82.51 | 51.57 | 82.51 | 51.57 |
| Microcrystalline Cellulose PH101 | 42.05 | 26.28 | 41.25 | 25.78 | 40.45 | 25.28 |
| croscarmellose sodium | 4.80 | 3.00 | 4.80 | 3.00 | 4.80 | 3.00 |
| colloidal silicon dioxide 200 | 1.60 | 1.00 | 1.60 | 1.00 | 1.60 | 1.00 |
| Magnesium stearate | 0.80 | 0.50 | 1.60 | 1.00 | 2.40 | 1.50 |
| Total | 160.00 | 100.00 | 160.00 | 100.00 | 160.00 | 100.00 |

### 2. Powder properties data

| **Test No.** | **Bulk Density (g/ml)** | **Tapped Density (g/ml)** | **Carr Index (%)** | **Angle of Repose (°)** |
|---|---|---|---|---|
| **4-1** | 0.499 | 0.753 | 33.73 | 41.05 |
| **4-2** | 0.488 | 0.742 | 34.23 | 40.35 |
| **4-3** | 0.496 | 0.756 | 34.39 | 40.28 |
| **4-4** | 0.490 | 0.720 | 31.94 | 38.0 |
| **4-5** | 0.500 | 0.720 | 30.56 | 37.2 |
| **4-6** | 0.510 | 0.720 | 29.17 | 36.4 |

### 3. Drug dissolution results

| **Test No.** | **Dissolution Item** | **Dissolution (%) (n=3)** | | | |
|---|---|---|---|---|---|
| | | **5 min** | **15 min** | **30 min** | **45 min** |
| **4-1** | Mean | 55 | 85 | 96 | 98 |
| | RSD% | 23.4 | 7.5 | 3.8 | 1.9 |
| **4-2** | Mean | 56 | 88 | 99 | 100 |
| | RSD% | 2.3 | 3.3 | 2.7 | 1.2 |
| **4-3** | Mean | 54 | 92 | 98 | 99 |
| | RSD% | 9.5 | 2.4 | 0.9 | 0.5 |
| **4-4** | Mean | 98.8 | 103.0 | 103.4 | 103.4 |
| | RSD% | 1.8 | 1.8 | 1.7 | 1.8 |
| **4-5** | Mean | 91.6 | 99.2 | 100.1 | 100.3 |
| | RSD% | 3.0 | 2.1 | 2.0 | 2.0 |
| **4-6** | Mean | 93.3 | 98.3 | 98.9 | 99.1 |
| | RSD% | 5.2 | 1.1 | 1.4 | 1.3 |

The powder properties data show that the three pharmaceutical compositions with different lubricant dosages all have good powder flowability, and the powder flowability tends to improve with the increase of lubricant dosage. The drug dissolution results show that the three pharmaceutical compositions with magnesium stearate in the range of 0.5% to 1.5% all have faster dissolution rates and good dissolution consistency in a dissolution medium (0.1 mol/L hydrochloric acid solution) with a pH of 1.2.

### Example 5

### (Evaluation of the effect of disintegrant)

### 1. Drug components and contents

According to the components and contents described in the following table, pharmaceutical compositions 5-1 to 5-6 were prepared by referring to the method of Example 1, and the dosage of the disintegrant was investigated.

| **Test No.** | **5-1** | | **5-2** | | **5-3** | |
|---|---|---|---|---|---|---|
| **Component and Content** | **Dosage mg** | **Percentage %** | **Dosage mg** | **Percentage %** | **Dosage mg** | **Percentage %** |
| API | 112.96 | 31.38 | 112.96 | 31.38 | 112.96 | 31.38 |
| Lactose Monohydrate T80 | 157.49 | 43.75 | 152.69 | 42.41 | 147.89 | 41.08 |
| Microcrystalline Cellulose PH102 | 78.75 | 21.87 | 76.35 | 21.21 | 73.95 | 20.54 |
| croscarmellose sodium | 3.60 | 1.00 | 10.80 | 3.00 | 18.00 | 5.00 |
| colloidal silicon dioxide 200 | 3.60 | 1.00 | 3.60 | 1.00 | 3.60 | 1.00 |
| Magnesium stearate | 3.60 | 1.00 | 3.60 | 1.00 | 3.60 | 1.00 |
| Total | 360.00 | 100.00 | 360.00 | 100.00 | 360.00 | 100.00 |
| | | | | | | |

| **Test No.** | **5-4** | | **5-5** | | **5-6** | |
|---|---|---|---|---|---|---|
| **Component and Content** | **Dosage mg** | **Percentage %** | **Dosage mg** | **Percentage %** | **Dosage mg** | **Percentage %** |
| API | 28.24 | 17.65 | 28.24 | 17.65 | 28.24 | 17.65 |
| Lactose Monohydrate 316 | 82.51 | 51.57 | 82.51 | 51.57 | 82.51 | 51.57 |
| Microcrystalline Cellulose PH101 | 44.45 | 27.78 | 41.25 | 25.78 | 38.05 | 23.78 |
| croscarmellose sodium | 1.60 | 1.00 | 4.80 | 3.00 | 8.00 | 5.00 |
| colloidal silicon dioxide 200 | 1.60 | 1.00 | 1.60 | 1.00 | 1.60 | 1.00 |
| Magnesium stearate | 1.60 | 1.00 | 1.60 | 1.00 | 1.60 | 1.00 |
| Total | 160.00 | 100.00 | 160.00 | 100.00 | 160.00 | 100.00 |

### 2. Powder properties data

| **Test No.** | **Bulk Density (g/ml)** | **Tapped Density (g/ml)** | **Carr Index (%)** | **Angle of Repose (°)** |
|---|---|---|---|---|
| 5-4 | 0.48 | 0.71 | 32.39 | 36.5 |
| 5-5 | 0.50 | 0.72 | 30.56 | 37.2 |
| 5-6 | 0.50 | 0.73 | 31.51 | 36.5 |

### 3. Drug dissolution results

| **Test No.** | **Dissolution Item** | **Dissolution (%) (n=3)** | | | |
|---|---|---|---|---|---|
| | | **5 min** | **15 min** | **30 min** | **45 min** |
| 5-1 | Mean | 42 | 90 | 98 | 99 |
| | RSD% | 7.0 | 2.1 | 0.6 | 0.4 |
| 5-2 | Mean | 56 | 88 | 99 | 100 |
| | RSD% | 2.3 | 3.3 | 2.7 | 1.2 |
| 5-3 | Mean | 53 | 92 | 99 | 100 |
| | RSD% | 28.8 | 5.1 | 2.3 | 1.1 |
| 5-4 | Mean | 85.8 | 99.6 | 100.2 | 100.5 |
| | RSD% | 9.8 | 2.7 | 2.5 | 2.4 |
| 5-5 | Mean | 91.6 | 99.2 | 100.1 | 100.3 |
| | RSD% | 3.0 | 2.1 | 2.0 | 2.0 |
| 5-6 | Mean | 96.3 | 100.0 | 100.7 | 101.0 |
| | RSD% | 1.3 | 1.5 | 1.4 | 1.4 |

The above experimental results show that the powder flowability is good when the content of croscarmellose sodium as a disintegrant is within the range of 1% to 5%. The different pharmaceutical compositions have a faster dissolution rate and good dissolution consistency in a dissolution medium (0.1 mol/L hydrochloric acid solution) with a pH of 1.2.

### Example 6

### (Development of small-scale preparations)

### 1. Drug components and contents

Based on the component ratios and test results of the 100 mg dosage form of pharmaceutical composition 5-2 from Example 5, the inventors prepared pharmaceutical compositions 6-1 and 6-2 in 5 mg and 25 mg dosage forms. While maintaining the same ratios of glidant and lubricant, the amount of diluent was adjusted to achieve the target fill weight. The manufacturing process followed the same procedure as Example 5.

| **Test No.** | **6-1** | | **6-2** | |
|---|---|---|---|---|
| Preparation Specification | 5 mg | | 25 mg | |
| component and content | Dosage mg | Percentage % | Dosage mg | Percentage % |
| API | 5.65 | 3.53 | 28.24 | 17.65 |
| Lactose Monohydrate T80 | 97.57 | 60.98 | 82.51 | 51.57 |
| Microcrystalline Cellulose PH102 | 48.78 | 30.49 | 41.25 | 25.78 |
| croscarmellose | 4.80 | 3.00 | 4.80 | 3.00 |
| sodium | | | | |
| colloidal silicon dioxide 200 | 1.60 | 1.00 | 1.60 | 1.00 |
| Magnesium stearate | 1.60 | 1.00 | 1.60 | 1.00 |
| Total | 160.00 | 100.00 | 160.00 | 100.00 |

### 2. Drug dissolution effect

| **Preparation Specification** | **Dissolution Item** | **Dissolution (%) (n=3)** | | | |
|---|---|---|---|---|---|
| | | **5 min** | **15 min** | **30 min** | **45 min** |
| 5 mg | Mean | 63 | 92 | 99 | 99 |
| | RSD% | 4.5 | 3.3 | 0.6 | 1.0 |
| 25 mg | Mean | 74 | 101 | 102 | 102 |
| | RSD% | 7.1 | 2.7 | 2.0 | 2.2 |

From the above test results, it can be seen that the pharmaceutical compositions in 5 mg and 25 mg small-scale dosage forms prepared using this prescription also have good dissolution behavior, which further illustrates that the prescription design is reasonable and meets the requirements of clinical research and drug marketing.

### Example 7

### (Prescription Development and Results)

### 1. Prescription composition

The present invention provides a pharmaceutical composition of a CSF-1R inhibitor, prepared into capsules of three specifications: 5 mg, 25 mg, and 100 mg. The excipients used and their proportions differ across the three formulations. The excipients employed include lactose monohydrate T80/lactose monohydrate 316, microcrystalline cellulose PH101/microcrystalline cellulose PH102, croscarmellose sodium, colloidal silicon dioxide 200, magnesium stearate, and empty gelatin capsules. The dosages of each component are detailed in the table below:

| **Test No.** | **7-1** | | **7-2** | | **7-3** | | **7-4** | |
|---|---|---|---|---|---|---|---|---|
| Prescription component | 5 mg specification | | 25 mg specification | | 100 mg specification | | 25 mg specification | |
| | mg/capsule | Proportion % | mg/capsule | Proportion % | mg/capsule | Proportion % | mg/capsule | Proportion % |
| Capsule contents | | | | | | | | |
| API | 5.65 | 3.53 | 28.24 | 17.65 | 112.96 | 31.38 | 28.24 | 17.65 |
| Lactose Monohydrate T80 | 97.57 | 60.98 | 82.51 | 51.57 | 152.69 | 42.41 | / | / |
| Lactose Monohydrate 316 | / | / | / | / | / | / | 82.51 | 51.57 |
| Microcrystalline Cellulose PH102 | 48.78 | 30.49 | 41.25 | 25.78 | 76.35 | 21.21 | / | / |
| Microcrystalline Cellulose PH101 | / | / | / | / | / | / | 41.25 | 25.78 |
| Croscarmellose sodium | 4.80 | 3.00 | 4.80 | 3.00 | 10.80 | 3.00 | 4.80 | 3.00 |
| colloidal silicon dioxide 200 | 1.60 | 1.00 | 1.60 | 1.00 | 3.60 | 1.00 | 1.60 | 1.00 |
| Magnesium stearate | 1.60 | 1.00 | 1.60 | 1.00 | 3.60 | 1.00 | 1.60 | 1.00 |
| Total | 160.00 | 100.00 | 160.00 | 100.00 | 360.00 | 100.00 | 160.00 | 100.00 |

| Capsule shell | | | | | | | | |
|---|---|---|---|---|---|---|---|---|
| empty gelatin capsule | 3#, opaque, rich yellow | | 3#, opaque, white | | 0#, opaque, white | | 3#, opaque, white | |
| Note | "/" indicates not included. | | | | | | | |

### 2. Preparation process

1) Weighing: The API (for 5 mg specification, it should be crushed in advance, 60 s), lactose monohydrate 316 (or lactose monohydrate T80), microcrystalline cellulose PH101 (or microcrystalline cellulose PH102), croscarmellose sodium, colloidal silicon dioxide 200, and magnesium stearate were weighed according to the prescribed amount.
2) Screening of API and excipients: The API and colloidal silicon dioxide 200 were sieved through a 60-mesh sieve for later use; microcrystalline cellulose PH101 (or microcrystalline cellulose PH102) was sieved through a 60-mesh sieve for later use; lactose monohydrate 316 (or lactose monohydrate T80) and croscarmellose sodium were sieved through a 40-mesh sieve for later use, separately; and magnesium stearate was sieved through a 30-mesh sieve for later use.
3) Main mixing: Except for magnesium stearate, the excipients and API were placed in a hopper mixer and blended for 20 minutes at a mixing speed of 20 rpm, yielding the main mixed powder.
4) Overall mixing: Magnesium stearate was added to the main mixed powder and mixed for 5 minutes at a mixing speed of 20 rpm to obtain a total mixed powder.
5) Discharging: The total mixed powder was removed and placed in an LDPE bag.
6) Capsule filling: The total mixed powder was loaded into the capsule filling machine and filled into opaque, rich yellow 3# capsules or 0# capsules.

### 3. Preparation analysis

| **Test No.** | | 7-1 | 7-2 | 7-3 |
|---|---|---|---|---|
| Key Quality Data | Content Uniformity (A+2.2S) | 3.2 | N/A | N/A |
| | Related Substances (Total Impurities, %) | 0.20 | 0.16 | 0.17 |
| | Dissolution (30 min, %) | 101.00 | 103.15 | 99.74 |
| Note | "N/A" indicates not detected. | | | |

| | | | | |
|---|---|---|---|---|
| Note: Take 10 capsules for content uniformity determination. | | | | |

### 4. Content uniformity study

The applicant of the present invention focused on studying the content uniformity of pharmaceutical compositions 7-2 and 7-4 during the overall mixing, discharging and capsule filling processes. The specific sampling time and method for content uniformity determination were as follows: 1) sampling 11 times for testing after overall mixing; 2) sampling and testing at the three stages of the discharging process: early stage, mid-term and late stage; 3) sampling and testing 2 to 3 capsules at least every 5 minutes during the capsule filling process. The specific measurement results are as follows.

### 4.1 Determination of content uniformity after overall mixing

| **Test No.** | **7-2** | | | **7-4** | | |
|---|---|---|---|---|---|---|
| sampling times | **Content (%)** | **Average (%)** | **RSD (%)** | **Content (%)** | **Average (%)** | **RSD (%)** |
| 1 | 100.2 | | | 100.2 | | |
| 2 | 100.0 | | | 101.4 | | |
| 3 | 100.0 | | | 100.3 | | |
| 4 | 101.3 | | | 100.2 | | |
| 5 | 98.4 | 99.8 | 0.9 | 100.3 | 100.3 | 0.4 |
| 6 | 100.5 | | | 99.9 | | |
| 7 | 100.1 | | | 100.3 | | |
| 8 | 99.8 | | | 100.3 | | |
| 9 | 98.1 | | | 99.6 | | |
| 10 | 99.4 | | | 100.1 | | |
| 11 | 100.3 | | | 100.4 | | |

### 4.2 Determination of content uniformity during discharging

| **Test No.** | **7-2** | | | **7-4** | | |
|---|---|---|---|---|---|---|
| **Sampling stage** | **Content (%)** | **Average (%)** | **RSD (%)** | **Content (%)** | **Average (%)** | **RSD (%)** |
| Early stage of the discharg | 103.7 | 100.6 | 3.3 | 100.7 | 100.6 | 0.71 |
| | 102.9 | | | 100.3 | | |
| Mid term of the discharg | 95.5 | | | 101.5 | | |
| | 102.2 | | | 101.3 | | |
| Late stage of the discharg | 97.6 | | | 99.8 | | |
| | 101.5 | | | 99.9 | | |

### 4.3 Determination of content uniformity during capsule filling process

| **Test No.** | **7-2** | | **7-4** | |
|---|---|---|---|---|
| **Measurement results** | **Average (%)** | **RSD(%)** | **Average (%)** | **RSD(%)** |
| | 103.3 | 6.8 | 100.4 | 1.1 |

The experimental results above indicate that pharmaceutical compositions 7-2 and 7-4 exhibit good uniformity after overall mixing. During discharge, pharmaceutical composition 7-4 demonstrates better content uniformity, while pharmaceutical composition 7-2 shows a tendency toward demixing. During capsule filling, pharmaceutical composition 7-4 maintains better content uniformity, whereas pharmaceutical composition 7-2 exhibits greater content fluctuations and noticeable demixing.

### 5. Stability studies

In order to investigate the rationality and stability of the capsule formulation prescriptions, preliminary influencing factors and stability studies were conducted on pharmaceutical compositions 7-1 to 7-4.

### 5.1 Stability samples

| **Test No.** | **7-1** | **7-2** | **7-3** | **7-4** |
|---|---|---|---|---|
| Quantity Specifications | 25 capsules/bottle | 25 capsules/bottle | 25 capsules/bottle | 35 capsules/bottle |
| Sample Packaging | high-density polyethylene bottle for oral solid medications, 45 mL | high-density polyethylene bottle for oral solid medications, 45 mL | high-density polyethylene bottle for oral solid medications, 75 mL | high-density polyethylene bottle for oral solid medications, 75 mL |
| | child-resistant cap for oral solid medications | child-resistant cap for oral solid medications | child-resistant cap for oral solid medications | child-resistant cap for oral solid medications |

### 5.2 Sample placement conditions

| **Storage conditions** | **10 days** | **30 days** | **6 monthes** |
|---|---|---|---|
| Light exposure (opened) | one bottle | N/A | N/A |
| 60 °C (opened) | one bottle | one bottle | N/A |
| 75%RH (opened) | one bottle | one bottle | N/A |
| 40 °C/75%RH (opened) | one bottle | one bottle | N/A |
| 40 °C/75%RH (closed) | N/A | one bottle | N/A |
| 30 °C/65%RH (closed) | N/A | one bottle | one bottle |
| Note | "N/A" indicates no sample was placed. | | |

### 5.3 Stability data

| **Test No.** | **Storage conditions** | **Dissolution (%) (n=3)** | | | | |
|---|---|---|---|---|---|---|
| | | **Dissolution Item** | **5 min** | **15 min** | **30 min** | **45 min** |
| | 0 day | Mean | 95 | 101 | 101 | 101 |
| | | RSD% | 2.9 | 0.5 | 0.6 | 0.8 |
| | 10 days 40 °C/75% RH (opened) | Mean | 94 | 98 | 99 | 99 |
| | | RSD% | 2.1 | 1.4 | 0.7 | 0.6 |
| 7-1 | 30 days 40 °C/75% RH (opened) | Mean | 84 | 98 | 98 | 98 |
| | | RSD% | 4.0 | 1.7 | 1.5 | 1.8 |
| | 30 days 40 °C/75% RH (closed) | Mean | 90 | 99 | 100 | 99 |
| | | RSD% | 5.0 | 1.6 | 1.3 | 1.3 |
| 7-2 | 0 day | Mean | 94 | 102 | 103 | 103 |
| | | RSD% | 4.3 | 1.5 | 1.6 | 1.7 |
| | 10 days 40 °C/75% RH (opened) | Mean | 82 | 98 | 100 | 100 |
| | | RSD% | 13.8 | 1.2 | 2.1 | 2.1 |
| | 30 days 40 °C/75% RH (opened) | Mean | 85 | 94 | 95 | 96 |
| | | RSD% | 6.0 | 2.4 | 1.8 | 1.6 |
| | 30 days 40 °C/75% RH (closed) | Mean | 76 | 95 | 98 | 99 |
| | | RSD% | 34.3 | 7.9 | 3.2 | 1.7 |
| 7-3 | 0 day | Mean | 52 | 96 | 100 | 100 |
| | | RSD% | 23.1 | 2.1 | 1.2 | 1.1 |
| | 10 days 40 °C/75% RH (opened) | Mean | 46 | 90 | 96 | 98 |
| | | RSD% | 11.5 | 6.8 | 1.9 | 0.9 |
| | 30 days 40 °C/75% RH (opened) | Mean | 53 | 83 | 91 | 95 |
| | | RSD% | 7.2 | 5.6 | 2.8 | 1.3 |
| | 30 days 40 °C/75% RH (closed) | Mean | 49 | 88 | 98 | 100 |
| | | RSD% | 19.8 | 2.9 | 0.7 | 0.2 |
| 7-4 | 0 day (n=6) | Mean | 86.4 | 99.4 | 101.0 | 101.3 |
| | | RSD% | 10.8 | 1.3 | 1.0 | 1.0 |
| | 6 monthes 30 °C/65% RH (closed) | Mean | 88.7 | 100.4 | 101.4 | 101.6 |
| | | RSD% | 4.5 | 1.0 | 0.7 | 0.7 |

The above data show that the accelerated conditions (40°C/75% RH or 30°C/65% RH) have no effect on the dissolution of capsules of various specifications.

### 5.4 Related substances (%)

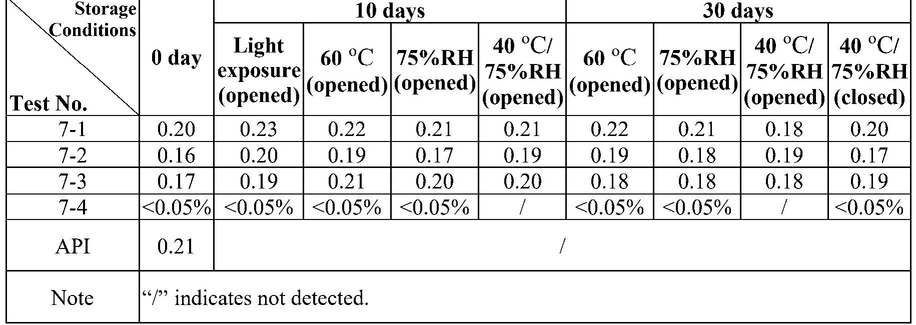

As indicated by the above data, when capsules of various specifications are subjected to various different conditions, the relevant substances show no increase and remain stable in nature.

### 5.5 Test conclusion

The present invention develops a quick-release capsule of a CSF-1R inhibitor, which can be prepared using a powder direct filling process, and the excipients used are all common excipients that are pharmaceutically acceptable. The key quality attributes of the 5mg, 25mg, and 100mg dosage forms produced under this invention all meet the release standards. Stability studies have shown that the product's physical properties (dissolution) and chemical properties are stable after storage for 30 days or even 6 months under accelerated conditions (40 °C/75% RH and 60 °C), meeting clinical pharmaceutical standards. Good stability supports its storage at room temperature.

This invention involves comprehensive and systematic research on the product, examining not only the types and contents of functional excipients but also the feasibility of key processes and parameter tolerance ranges. The powder direct filling process employed in this invention offers advantages such as fewer production steps, lower time and labor costs, and simplicity for scale-up, enabling the production of uniform, stable formulations with excellent release performance.

All documents mentioned in the present invention are incorporated by reference in this application, just as each document is individually cited. In addition, it should be understood that various modifications or changes may be made by those skilled in the art after reading the above disclosed content of the present invention, and these equivalent forms also fall within the scope defined by the claims appended hereto.

## Claims

1. A pharmaceutical composition comprising a free base or an acid salt of a compound of formula (I) as an active ingredient, a pharmaceutically acceptable diluent, and other pharmaceutically acceptable carrier, 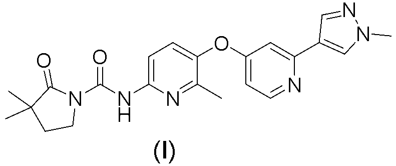 wherein the other pharmaceutically acceptable carrier is one or more of a glidant, a lubricant, and a disintegrant.

2. The pharmaceutical composition according to claim 1, wherein the acid salt of the compound of formula (I) is selected from hydrochloride, sulfate, hydrobromide, hydrofluoride, hydroiodide, phosphate, acetate, dichloroacetate, trichloroacetate, trifluoroacetate, benzenesulfonate, p-toluenesulfonate, 4-chlorobenzenesulfonate, 1,5-naphthalene disulfonate, naphthalene-2-sulfonate, ethane-1,2-disulfonate, methanesulfonate, ethanesulfonate, benzoate, decanoate, hexanoate, caprylate, cinnamate, citrate, cyclohexanesulfamate, camphorsulfonate, aspartate, camphorate, gluconate, glucuronate, glutamate, isoascorbate, lactate, aspartate, malate, mandelate, pyroglutamate, tartrate, lauryl sulfate, dibenzoyltartrate, formate, fumarate, galactonate, gentisate, acetohydroxamate, malonate, succinate, glutarate, adipate, sebacate, 2-ketoglutarate, glycolate, hippurate, isethionate, lactobionate, ascorbate, aspartate, laurate, camphorate, maleate, nicotinate, oleate, orotate, oxalate, palmitate, pamoate, propionate, 4-acetamidobenzoate, 4-aminobenzoate, salicylate, 4-aminosalicylate, 2,5-dihydroxybenzoate, 1-hydroxy-2-naphthoate, stearate, thiocyanate, undecylenate, or succinate;
preferably, the acid salt of the compound of formula (I) is selected from hydrochloride, sulfate, phosphate, methanesulfonate, citrate, malate, fumarate or tartrate.

3. The pharmaceutical composition according to claim 1 or 2, wherein the active ingredient is an anhydrate, hydrate or solvate of the free base or acid salt of the compound of formula (I);
preferably, the active ingredient is a solvate of the free base or acid salt of the compound of formula (I), and the solvent is selected from alcohols, chloroalkanes, ketones, ethers, cyclic ethers, esters, alkanes, cycloalkanes, benzenes, amides, sulfoxides or a mixture thereof, or an aqueous solution thereof;
more preferably, the active ingredient is a solvate of the free base or acid salt of the compound of formula (I), and the solvent is methanol, ethanol, n-propanol, isopropanol, dichloromethane, acetonitrile, acetone, 1,4-dioxane, tetrahydrofuran, N,N-dimethylformamide, ethyl acetate, isopropyl acetate, methyl tert-butyl ether, 2-methoxyethyl ether or a mixture thereof, or an aqueous solution thereof;
preferably, the active ingredient is an anhydrate or hydrate of the free base or acid salt thereof;
more preferably, the active ingredient is an anhydrate of the free base or acid salt thereof;
more preferably, the active ingredient is a hydrate of the free base or acid salt thereof, wherein each molecule of the hydrate contains 1 to 3 water molecules;
more preferably, the active ingredient is a hydrate of the free base or acid salt thereof, wherein each molecule of the hydrate contains one water molecule.

4. The pharmaceutical composition according to claim 1, wherein the content of the active ingredient is calculated as a hydrochloride monohydrate of the compound of formula (I), and the pharmaceutical composition contains 0.1% to 60.0% w/w of the active ingredient relative to the total weight of the composition;
preferably, the pharmaceutical composition contains 0.1% to 40.0% w/w of the active ingredient relative to the total weight of the composition;
more preferably, the pharmaceutical composition contains 3.0% to 32.0% w/w of the active ingredient relative to the total weight of the composition.

5. The pharmaceutical composition according to claim 1, wherein the diluent is one or more of lactose, lactose hydrate and microcrystalline cellulose;
in particular, the pharmaceutical composition contains 40.0% to 99.9% w/w of the diluent relative to the total weight of the composition;
preferably, the pharmaceutical composition contains 50.0% to 94.0% w/w of the diluent relative to the total weight of the composition;
more preferably, the pharmaceutical composition contains 60.0% to 92.0% w/w of the diluent relative to the total weight of the composition.

6. The pharmaceutical composition according to claim 5, wherein the diluent is a mixture of lactose monohydrate and microcrystalline cellulose, and the mass ratio w/w of the lactose monohydrate and microcrystalline cellulose is 1:5 to 5:1; preferably, the mass ratio w/w of the lactose monohydrate and microcrystalline cellulose is 1:3 to 3:1; more preferably, the mass ratio w/w of the lactose monohydrate and microcrystalline cellulose is 1:1 to 3:1.

7. The pharmaceutical composition according to claim 6, wherein the lactose monohydrate is lactose monohydrate T80 or lactose monohydrate 316; and the microcrystalline cellulose is microcrystalline cellulose PH102 or microcrystalline cellulose PH101.

8. The pharmaceutical composition according to claim 7, wherein the diluent is a mixture of lactose monohydrate T80 and microcrystalline cellulose PH102, and the mass ratio w/w of the lactose monohydrate T80 and microcrystalline cellulose PH102 is 1:5 to 5:1; preferably, the mass ratio w/w of the lactose monohydrate T80 and microcrystalline cellulose PH102 is 1:3 to 3:1; more preferably, the mass ratio w/w of the lactose monohydrate T80 and microcrystalline cellulose PH102 is 1:1 to 3:1.

9. The pharmaceutical composition according to claim 7, wherein the diluent is a mixture of lactose monohydrate 316 and microcrystalline cellulose PH101, and the mass ratio w/w of the lactose monohydrate 316 and microcrystalline cellulose PH101 is 1:5 to 5:1; preferably, the mass ratio w/w of the lactose monohydrate 316 and microcrystalline cellulose PH101 is 1:3 to 3:1; more preferably, the mass ratio w/w of the lactose monohydrate 316 and microcrystalline cellulose PH101 is 1:1 to 3:1.

10. The pharmaceutical composition according to claim 1, wherein the glidant is one or more of colloidal silicon dioxide, precipitated silicon dioxide and talc;
preferably, the glidant is colloidal silicon dioxide;
more preferably, the glidant is colloidal silicon dioxide 200;
in particular, the pharmaceutical composition contains 0.1% to 10.0% w/w of the glidant relative to the total weight of the composition;
preferably, the pharmaceutical composition contains 0.5% to 5.0% w/w of the glidant relative to the total weight of the composition;
more preferably, the pharmaceutical composition contains 1.0% to 3.0% w/w of the glidant relative to the total weight of the composition.

11. The pharmaceutical composition according to claim 1, wherein the lubricant is one or more of magnesium stearate, sodium stearyl fumarate, glyceryl dibehenate, colloidal silicon dioxide, talc, other hydrogenated vegetable oils and triglyceride;
preferably, the lubricant is one or more of magnesium stearate, sodium stearyl fumarate, and glyceryl dibehenate;
more preferably, the lubricant is magnesium stearate or sodium stearyl fumarate;
in particular, the pharmaceutical composition contains 0.1% to 5.0% w/w of the lubricant relative to the total weight of the composition;
preferably, the pharmaceutical composition contains 0.5% to 2.0% w/w of the lubricant relative to the total weight of the composition;
more preferably, the pharmaceutical composition contains 0.5% to 1.5% w/w of the lubricant relative to the total weight of the composition.

12. The pharmaceutical composition according to claim 1, wherein the disintegrant is one or more of croscarmellose sodium, cross-linked polyvinyl pyrrolidone, sodium starch glycolate and analogue thereof;
preferably, the disintegrant is croscarmellose sodium;
in particular, the pharmaceutical composition contains 0.1% to 10.0% w/w of the disintegrant relative to the total weight of the composition;
preferably, the pharmaceutical composition contains 0.5% to 5.0% w/w of the disintegrant relative to the total weight of the composition;
more preferably, the pharmaceutical composition contains 1.0% to 5.0% w/w of the disintegrant relative to the total weight of the composition.

13. The pharmaceutical composition according to claim 1, wherein the pharmaceutical composition comprises the active ingredient and the diluent, the mass ratio w/w is (1.0 to 50.0): (50.0 to 94.0), and the content of the active ingredient is calculated as the hydrochloride monohydrate of the compound of formula (I); the diluent is lactose monohydrate and microcrystalline cellulose, and the mass ratio w/w is 1:1 to 3:1.

14. The pharmaceutical composition according to claim 1, wherein the pharmaceutical composition comprises the active ingredient and the diluent, the mass ratio w/w is (1.0-50.0): (60.0-92.0), and the content of the active ingredient is calculated as the hydrochloride monohydrate of the compound of formula (I); the diluent is lactose monohydrate T80 and microcrystalline cellulose PH102, and the mass ratio w/w is 1:1-3:1.

15. The pharmaceutical composition according to claim 1, wherein the pharmaceutical composition comprises the active ingredient and the diluent, the mass ratio w/w is (1.0-50.0): (60.0-92.0), and the content of the active ingredient is calculated as the hydrochloride monohydrate of the compound of formula (I); the diluent is lactose monohydrate 316 and microcrystalline cellulose PH101, and the mass ratio w/w is 1:1-3:1.

16. The pharmaceutical composition according to any one of claims 13 to 15, wherein the pharmaceutical composition further comprises colloidal silicon dioxide as a glidant, the content thereof is 0.5% to 5.0% w/w relative to the total weight of the composition; preferably 1.0% to 3.0% w/w; and/or
the pharmaceutical composition further comprises magnesium stearate or sodium stearyl fumarate as a lubricant, the content thereof is 0.5% to 2.0% w/w relative to the total weight of the composition; preferably 0.5% to 1.5% w/w; and/or
the pharmaceutical composition further comprises croscarmellose sodium as a disintegrant, the content thereof is 0.5% to 5.0% w/w relative to the total weight of the composition; preferably 1.0% to 5.0% w/w.

17. The pharmaceutical composition according to claim 1, wherein the pharmaceutical composition is in a capsule form.

18. The pharmaceutical composition according to claim 17, wherein the unit dosage form of the capsule contains 1 mg to 500 mg of active ingredient, and the content of the active ingredient is calculated as the free base of the compound of formula (I);
preferably, the unit dosage form contains 1 mg to 200 mg of active ingredient, the content of the active ingredient is calculated as the free base of the compound of formula (I);
more preferably, the unit dosage form contains 1 mg, 5 mg, 25 mg, 50 mg, 75 mg, 100 mg, 150 mg or 200 mg of active ingredient, and the content of the active ingredient is calculated calculated as the free base of the compound of formula (I).

19. A method for preparing the pharmaceutical composition according to claim 1, comprising the following steps:
step A: pre-treating the free base or acid salt of the compound of formula (I) and the glidant by sieving, either separately or in combination;
pre-treating the diluent, disintegrant and lubricant by sieving, either separately or in combination; 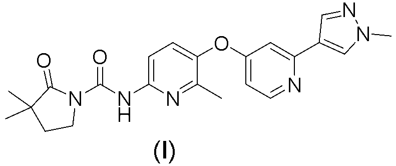
step B: uniformly mixing the mixture of the free base or acid salt of the compound of formula (I) and the glidant prepared in step A with the pretreated diluent and disintegrant, and placing them in a hopper mixer for mixing;
step C: uniformly mixing the mixed material prepared in step B and the pretreated lubricant, and placing them in a mixing barrel for mixing;
optionally, step D: filling the total mixed powder prepared in step C into capsules.

20. The preparation method according to claim 19, wherein the free base or acid salt of the compound of formula (I) and the glidant are mixed and then pretreated through a 50-80 mesh sieve; and/or
the diluent is independently pretreated through a 30-80 mesh sieve; and/or
the disintegrant is independently pretreated through a 30-50 mesh sieve; and/or
the lubricant is independently pretreated through a 20-40 mesh sieve.

21. The preparation method according to claim 19, wherein the free base or acid salt of the compound of formula (I) in step A is pulverized.

22. Use of the pharmaceutical composition according to any one of claims 1 to 18 in the manufacture of a CSF-1R inhibitor drug.

23. Use of the pharmaceutical composition according to any one of claims 1 to 18 in the manufacture of a medicament for treating a tumor, autoimmune disease, metabolic disease or metastatic disease associated with CSF1-R; preferably, the tumor is cancer.

24. The use according to claim 23, wherein the tumor, autoimmune disease, metabolic disease or metastatic disease associated with CSF1-R is a drug for ovarian cancer, pancreatic cancer, prostate cancer, lung cancer, breast cancer, kidney cancer, liver cancer, cervical cancer, bone metastatic cancer, papillary thyroid cancer, non-small cell lung cancer, colon cancer, gastrointestinal stromal tumor, solid tumor, melanoma, mesothelioma, glioblastoma, osteosarcoma, multiple myeloma, hyperproliferative disease, metabolic disease, neurodegenerative disease, metastasis of primary tumor site, myeloproliferative disease, leukemia, rheumatism arthritis, rheumatoid arthritis, osteoarthritis, multiple sclerosis, autoimmune nephritis, lupus, Crohn's disease, asthma, chronic obstructive pulmonary disease, osteoporosis, hypereosinophilic syndrome, mastocytosis or mast cell leukemia.

25. The pharmaceutical composition according to any one of claims 1 to 18 for use as a medicament for treating a tumor, autoimmune disease, metabolic disease or metastatic disease associated with CSF1-R.
